# EUROPÄISCHE PATENTANMELDUNG

(11) **EP 2 487 615 A2**
(43) Veröffentlichungstag der Anmeldung: **15.08.2012**
(21) Anmeldenummer: 12154009.0
(22) Anmeldetag: 06.02.2012
(51) Int. Cl.: G06F 19/00

(54) **System und Verfahren zur Nutzen- und Schadenbewertung medizinischer Interventionen und Medikamenten-Verabreichungen**

(30) Priorität: 11.02.2011 CH 2402011
(71) Anmelder: Mobileman GmbH, 8002 Zürich (CH)
(72) Erfinder: Ekan, Yusuf, 8002 Zürich (CH); Walser, Barbara, 8143 Stallikon (CH); Ciobanu, Daniel, 30163 Hannover (DE)
(74) Vertreter: Felber, Josef

(57) **Zusammenfassung**

Das vorliegende System und Verfahren bezieht sich auf die technische Anwendung zum Zwecke der Nutzen- und Schadenbewertung von medizinischen Interventionen, insbesondere zu deren technischer Nutzung durch die Beteiligten des Gesundheitssystems zur effizienteren und präziseren Nutzen- und Schadenbewertung von medizinischen Interventionen. Durch die erleichterte Dokumentations- und Wiedergabemöglichkeit der relevanten, teilweise unzähligen und komplexen Informationen seitens der durch die Krankheit eingeschränkten Patienten, wird der Krankheitsverlauf mittels eines Zentralcomputers mit Prozessor (15) und Datenbankserver (26) technisch über Eingabe- und Anzeigegeräte (2,20) so erfasst, dass eine erfolgreiche Therapie gefördert wird, da das vorliegende System und Verfahren in Verbindung mit der medizinischen Intervention einer chronischen oder auch temporären Krankheit eine lückenlose Krankheitsverfolgung mit Nutzen- und Schadenbewertung der medizinischen Intervention für die Patienten (1), die behandelnden Ärzte (23), die Krankenkassen (21) und die Arzneimittelhersteller (17) je nach Zugriffsberechtigung ermöglicht.

## Beschreibung

Die Erfindung betrifft ein computerimplementiertes System und Verfahren zur Nutzen- und Schadenbewertung von medizinischen Interventionen und Medikamenten-Verabreichungen.

Das vorliegende System und Verfahren bezieht sich auf die technische Anwendung zum Zwecke der Nutzen- und Schadenbewertung von medizinischen Interventionen und Medikamenten-Verabreichungen, insbesondere zu deren technischer Nutzung durch die Beteiligten des Gesundheitssystems zur effizienteren und präziseren Nutzen- und Schadenbewertung von medizinischen Interventionen.

Nach dem heutigen Stand der Technik stellen behandelnde Ärzte (Leistungserbringer der gesetzlichen Krankenkassen) nach einer oder mehreren Untersuchungen eines Betroffenen fest, ob dieser eine chronische Krankheit hat. Für die Behandlung von chronischen Krankheiten sind langwierige, jahrelange gar lebenslange Behandlungen nötig. Bei der Behandlung von chronischen Betroffenen werden diese oft nur in grossen Zeitabständen vom behandelnden Arzt konsultiert. Der Arzt stellt dann jeweils die Krankheitsaktivität per Stichtag fest. Auf Basis dieser Informationen verordnet und verschreibt er eine oder mehrere Medikamente (zusammenfassend als medizinische Intervention bezeichnet).

In den meisten Fällen der chronischen Krankheiten interveniert der Arzt durch die Verschreibung von verschreibungspflichtigen pharmazeutischen Präparaten (Arzneimittel), die auf Grundlage der evidenzbasierten Medizin (ebM) in klinischen Studien festgehalten wurden. In wiederkehrenden Konsultationen vom Betroffenen in der Arztpraxis in grösseren Zeitabständen evaluiert der Arzt den Nutzen und Schaden der medizinischen Intervention durch seine Untersuchungen. Dabei hält er Rücksprache mit dem Betroffenen über die vergangene Zeit seit der letzten Konsultation in Bezug auf die Lebensqualität, Beschwerden, Medikationen und Co-Medikationen sowie weitere Bewertungs- und Untersuchungsfragen rund um den Nutzen und den Schaden der medizinischen Intervention. Bei Bedarf hält er zusätzlich Rücksprache mit weiteren Fachpersonen.

Die Nutzen- und Schadenbewertung wird bereits vor Zulassung eines pharmazeutischen Präparates auf Evidenzbasis in klinischen Studien durchgeführt. Eine klinische Studie wird oft mit Prüfern aus kontrollierten Teilpopulationen oder gesunden Probanden in einem artifiziellen Umfeld durchgeführt, um Medikamente auf deren Wirksamkeit und Sicherheit zu überprüfen. Diese Studien können zum Beispiel in Deutschland gemäss dem Arzneimittelmarktneuordnungsgesetz (AMNOG), Stand 1.1.2011, mit zusätzlichen Nutzen- und Schadenbewertungsparametern ergänzt und entsprechend über die Markteinführung hinaus fortgeführt werden, um den therapeutischen Zusatznutzen der Präparate gegenüber den staatlichen und privaten Institutionen unter Alltagsbedingungen zu belegen. Durch die positive Darlegung der Nutzen- und Schadenbewertung wird dem Hersteller von Medikamenten der erstattungsberichtigte Markt- und Vertriebszugang ermöglicht. Von zentraler Bedeutung ist hier die zunehmende Belastung von Gesundheitsbudgets, die nachhaltig nicht mehr tragbar durch die immer älter werdende Bevölkerung sind und den daraus zusammenhängenden, steigenden Gesundheitskosten.

Es bestehen zwar technische Lösungen zur Überwachung von Krankheitsverläufen. Diese weisen aber weder einen technisch spezifischen Bezug noch eine gezielte Verwendung zur Nutzen- und Schadenbewertung von medizinischen Interventionen aus.

Aus der EP 1 972 270 sind ein Verfahren und Glukoseüberwachungs-system zur Überwachung individueller Stoffwechselreaktionen bekannt. Bei dem vorgeschlagenen Verfahren handelt es sich um eine Messung, Verarbeitung und Analyse des Blutzuckerspiegels. Hierbei werden die medizinischen Interventionen nicht auf die Nutzen- und Schadenbewertung von Medikamenten hin analysiert, sondern auf die Aufnahme und Verarbeitung von metabolischen Antworten und auf das Langzeitmanagement von dietätischen Behandlungen.

Aus der EP 1 788 499 ist eine Vorrichtung zur automatisierten Optimierung von Behandlungsplänen bekannt. Diese dient dem Zweck der Optimierung von Behandlungen. Die medizinische Intervention wird auch hier nicht auf die Nutzen- und Schadenbewertung von Medikamenten hin analysiert und ausgewertet.

Daher lässt sich feststellen, dass die bestehenden technischen Lösungen zur Datenerhebung weder einen technisch spezifischen Bezug und noch eine gezielte Verwendung zur Nutzen- und Schadenbewertung von medizinischen Interventionen ausweisen.

Aus der EP 1 351 181 sind ein Computersystem und Verfahren zur Datenerfassung für die Ermittlung des Verlaufs einer chronischen Erkrankung bekannt. Diese Erfindung dient zwar vorwiegend der verbesserten Benutzerfreundlichkeit, jedoch wird dem Patienten nicht die Möglichkeit eröffnet, die Datenerhebung in Abhängigkeit zu seinem spezifischen Krankheitsbild und -stadium durchzuführen. So sieht die eben benannte Erfindung keine technische Eingabemöglichkeit der Daten seitens des Patienten in Abhängigkeit zu seiner Krankheit und der daraus folgenden spezifischen körperlichen Einschränkung, wie etwa durch text- und zeichenbasierend und/oder Ton-/Bild-/Videoaufnahmen.

Nach dem Stand der Technik kann der Patient das von ihm eingenommene Medikament mit der dazugehörigen Dosierung und Nebenwirkung per Hand manuell in einem Medikationstagebuch dokumentieren. Zusätzlich zu den vorangehend angeführten Nachteilen entsteht hier noch die nachteilige Möglichkeit, dass aufgrund der bisherigen Eingabemöglichkeiten, der Patient unvollständige oder fehlerhafte Daten über das von ihm eingenommene Medikament dokumentiert. Eine korrekte Nutzen- und Schadenbewertung wird somit zumindest erschwert.

Durch die erleichterte Dokumentations- und Wiedergabemöglichkeit der relevanten, teilweise unzähligen und komplexen Informationen, seitens der durch die Krankheit eingeschränkten Patienten, wird der Krankheitsverlauf technisch so erfasst, dass eine erfolgreiche Therapie gefördert wird, da das vorliegende System und Verfahren in Verbindung mit der medizinischen Intervention einer chronischen Krankheit für den Patienten und den behandelnden Arzt eine lückenlose Krankheitsverfolgung mit Nutzen- und Schadenbewertung der medizinischen Intervention ermöglicht.

Betrachtet man die aus dem Zusammenspiel der eben, benannten einzelnen, bestehenden Nachteilen, so kann sich daraus eine fehleranfällige Nutzen- und Schadenbewertung von medizinischen Interventionen im Einsatz von medizinischen Präparaten (Arzneimittel) ergeben, die durch die bisherigen technischen Lösungen noch nicht behoben werden konnten.

Die Aufgabe der vorliegenden Erfindung besteht nun darin, ein computerimplementiertes, interaktives System und ein Verfahren zur Nutzen- und Schadenbewertung zu entwickeln, sodass die umfassenden relevanten Daten durch das erfinderische System und Verfahren verarbeitet, ausgewertet und effizient und mit allen Beteiligten ausgetauscht werden, zur Nutzen- und Schadenbewertung von medizinischen Interventionen. Abzugrenzen und hervorzuheben ist dabei, dass das vorliegende System weder eine eigenständige Therapie darstellt, noch Therapievorschläge erzeugen soll.

Die Aufgabe wird mit dem System nach Anspruch 1 sowie dem Verfahren nach Anspruch 2 gelöst. Vorteilhafte Ausgestaltungen und Weiterbildungen des Systems und Verfahrens sind Gegenstand der abhängigen Ansprüche.

Die Erfindung wird anhand eines Ausführungsbeispiels, welches in den Zeichnungen dargestellt ist, näher erläutert.
Es zeigt:
- Fig. 1: Eine schematische Repräsentation eines erfinderischen Systems zur Nutzen- und Schadenbewertung einer medizinischen Intervention;
- Fig. 2: Flussdiagramm, welches die erfinderische Datenakkumulation in Echtzeit durch den Patienten darstellt;
- Fig. 3: Flussdiagramm, welches die erfinderische Datenakkumulation in Echtzeit durch den Arzt darstellt;
- Fig. 4: Patientendateneingabe- und -anzeigegerät mit berührungsempfindlichem Bildschirm;
- Fig. 5: Arztdateneingabe- und -anzeigegerät mit berührungsempfindlichem Bildschirm;
- Fig. 6: Statistisches Berechnungsverfahren zur Nutzen- und Schadenbewertung von medizinischen Interventionen;

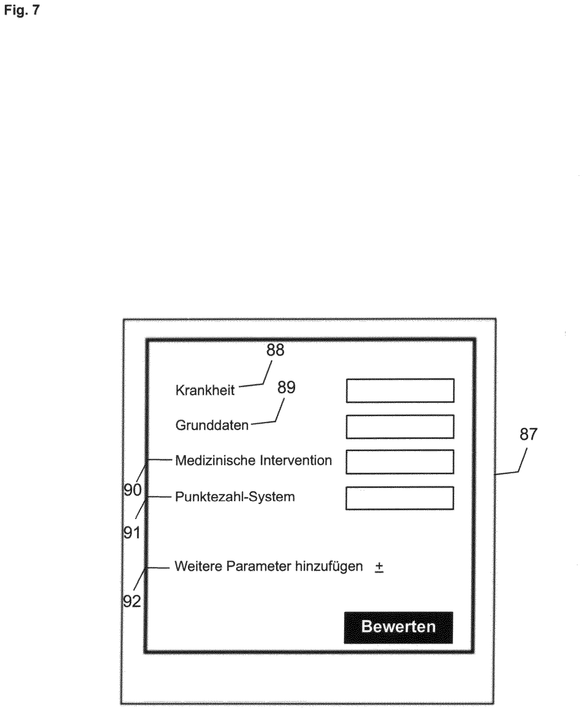

- Fig. 7: Nutzeroberfläche des Arztdateneingabe- und -anzeigegerät für die Einstellung der Grundparameter der Nutzen- und Schadenbewertung seitens des Arztes;
- Fig. 8: Eine grafische Nutzerschnittstelle für den Vergleich der Nutzen- und Schadenbewertung mehrerer medizinischer Interventionen für dieselbe Krankheit.

Alle Nutzer des computerimplementierten, interaktiven Systems und Verfahrens sind an einen Datenbankserver, das einen oder mehrere Server beinhaltet, elektronisch verbunden. Der Datenbankserver kann durch eine oder mehrere Programmspeichereinrichtungen wie z. B. eine CD-ROM (Compact Disc Read-Only Memory) programmiert werden, welches den computerlesbaren Programmcode beinhaltet.

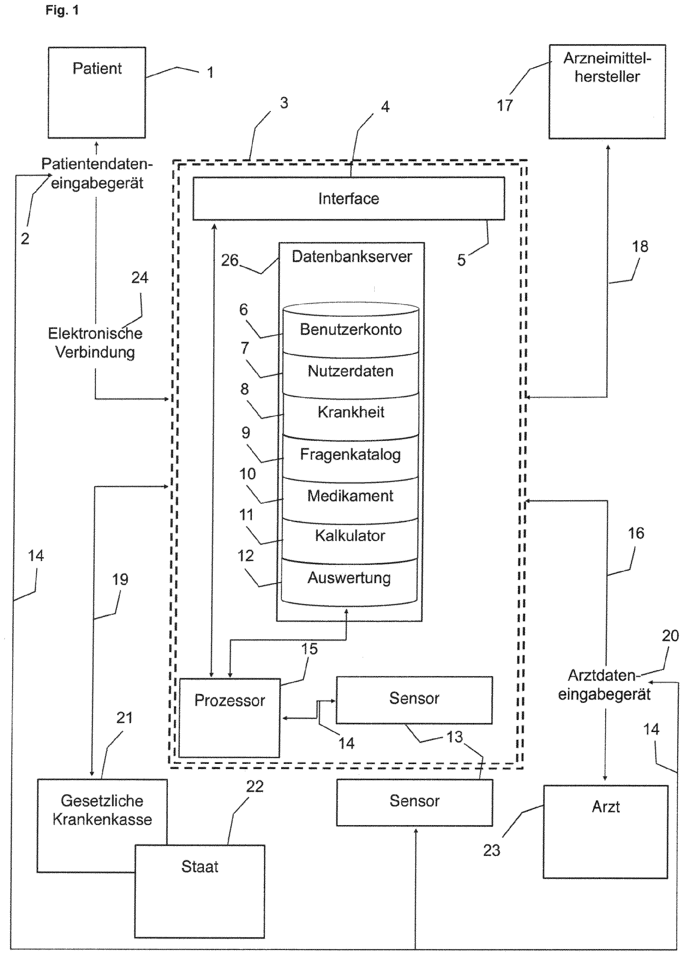

Das Gesamtkonzept erschliesst sich aus Figur 1: Das manuell verwendete Patienten- 2 sowie Arztdateneingabegerät 20 mit berührungsempfindlichem Bildschirm und/oder Tastatur können stationäre oder bewegliche Vorrichtungen sein, welche sich elektronisch 24, 16 über eine standardisierte Netzwerkschnittstelle oder Antenne 4, Interface 5 und Prozessor 15 zum Datenbankserver 26 verbinden.

Die manuell verwendeten Patienten- sowie Arztdateneingabegeräte können mit Zusatzvorrichtungen wie Sensorgeräten 13 oder mit weiteren internet- oder netzwerkfähigen Endgeräten unterstützt werden. Die Übermittlung 14 der Gesundheitsdaten kann beispielsweise, aber nicht abschliessend, mithilfe der Mobilfunk-, Sensor-, Infrarot- und/oder RFID-Technik (Radio Frequency Identification) zu den Eingabevorrichtungen und/oder zum Prozessor 15 erfolgen.

Weiterer Bestandteil des erfindungsgemässen System und Verfahren für die Nutzen- und Schadenbewertung besteht darin, dass der Patient 1 täglich oder wiederkehrend, aufgefordert über den programmierten Datenbankserver, über das Patientendateneingabe- und Kommunikationsgerät (Eingabevorrichtung) relevante medizinische Patientendaten in prozessorgenerierten Eingabeformularen 25 - 36 erfasst und diese in den Speicher eines zentralen Datenbankservers übermittelt, um sie schliesslich einer Nutzen- und Schadenbewertung 11, 12 zu unterziehen. Dabei unterscheidet die Erfindung zwischen einmaligen und wiederkehrenden Datenerhebungen. Der Arzt 23 erfasst die relevanten medizinischen Patientendaten wiederkehrend bei der Arztkontrolle über das Arztdateneingabe- und Kommunikationsgerät (Eingabevorrichtung) in prozessorgenerierten Eingabeformularen und übermittelt diese elektronisch an den Datenbankserver. Diese Daten kann der Arzt mit den vom Patienten selbsterhobenen Daten vergleichen und auswerten, um die Korrelation der von ihm vorgenommenen medizinischen Intervention im Rahmen der Nutzen- und Schadenbewertung mit den Ergebnissen der selbsterfassten Patientendaten zu überprüfen und zu beeinflussen.

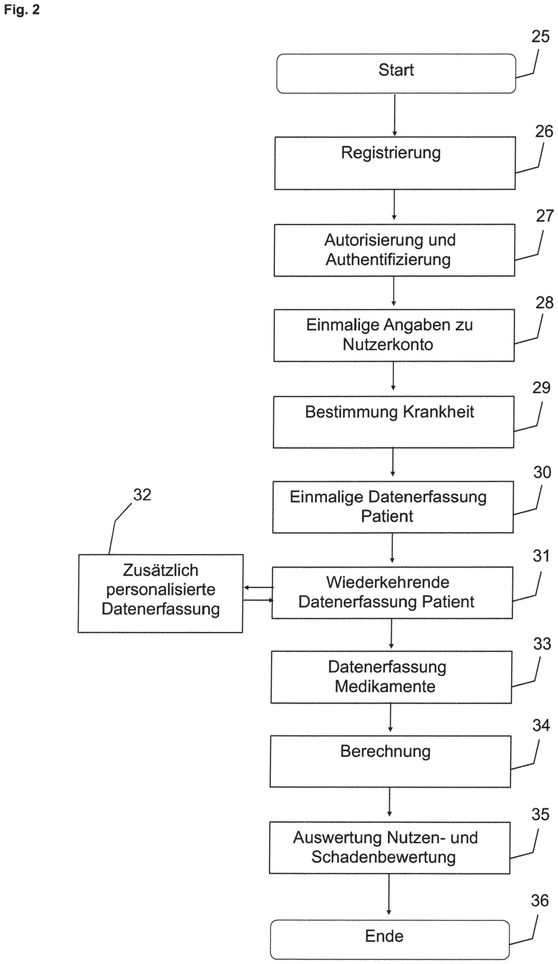

Eine einmalige, prozessorgenerierte Datenerhebung 30 in Figur 2 umfasst nach Registrierung 26 und Autorisierung 27 die Erstellung eines persönlichem und eindeutig zuordenbarem Nutzerkonto 28 im Speicher des Datenbankservers unter Angabe seiner Grundparameter wie Benutzername, Passwort, Alter, Geschlecht, Wohnort, Schulbildung, Herkunft, medizinisch-historische Daten, wie Datum der Symptome und Diagnose nach Krankheit, bisher eingenommene Medikamente sowie die Krankheit 29, die aus einer Liste ausgewählt wird, die vorausgehend aus den Informationen der öffentlichen Datenbank der World Health Organization (WHO) nach der International Classification of Diseases (ICD) kompiliert wurde, sowie weitere medizinisch relevante Daten.

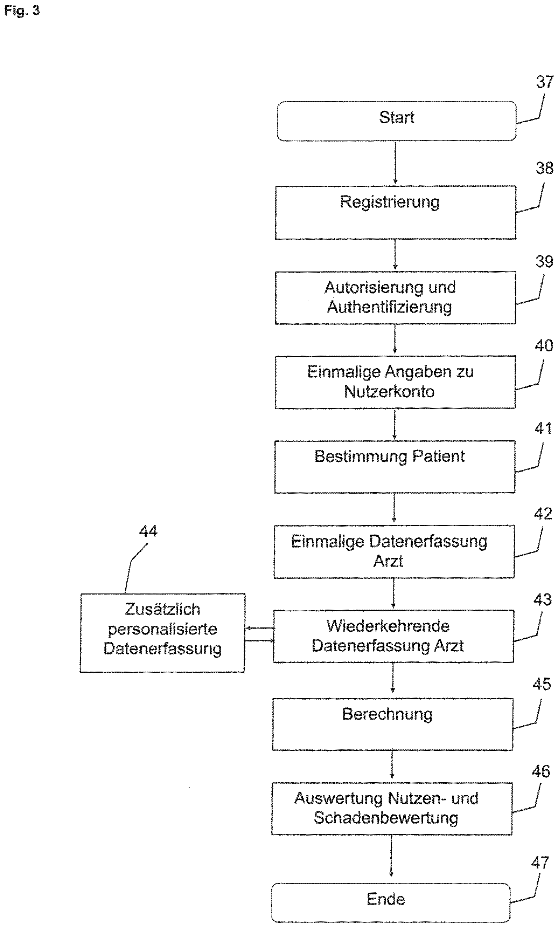

Die wiederkehrenden, prozessorgenerierten Datenerhebungen vom Patienten 32 und vom Arzt 44 in Figur 3 basieren auf standardisierten Fragenkatalogen, die je nach Krankheit variieren können. Die Datenerhebung mit Fragenkatalogen umfasst generelles und konkretes Befinden, wie beispielsweise, aber nicht abschliessend, seelisches, psychologisches sowie physiologisches Befinden. Die Fragestellungen des Fragenkatalogs wurden vorausgehend nach international anerkannten Kriterienkatalogen oder Health Assessment Questionnaires (HAQ) nach Krankheit für die numerische Erfassung der Krankheitsaktivität im Speicher des Datenbankservers eingegeben, welche die Grundlage sind für die Punktezahl-Systeme zur statistischen Erfassung von Krankheitsaktivitäten.

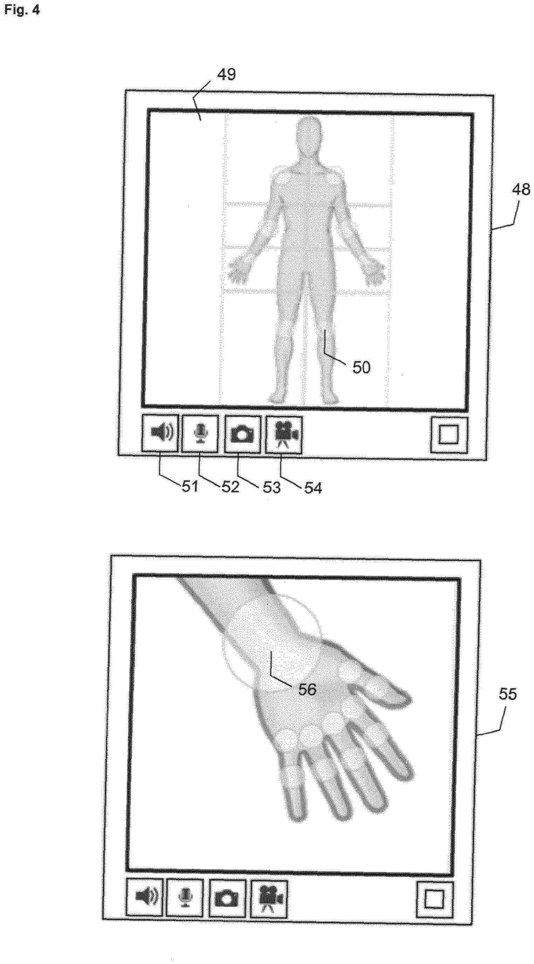

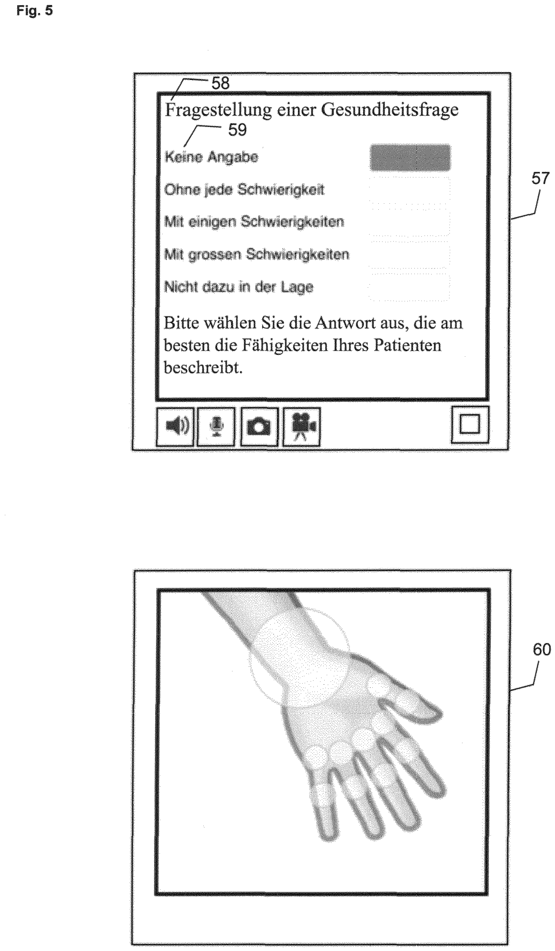

Die der Datenerhebung zugrunde liegenden Fragen sind dabei text- 57, 58, 59 in Figur 5 und oder zeichenbasierend 48 und 55 in Figur 4, die vorausgehend im Datenbankserver gespeichert wurden und durch den Datenbankserver generiert werden. Die zeichenbasierenden Fragen werden anhand von interaktiven Bildern menschlicher Anatomie 60 in Figur 5 abgebildet, bei der der Patient durch Berührung des Bildschirms 49 in Figur 4, durch Eingabe der Tastatur und/oder durch Eingabe eines Steuergeräts seine Parameter in das interaktive Bild 50, 56 einpflegen kann.

Zusätzlich kann die Datenerhebung auf Grundlage von visuellen und akustischen Daten, wie etwa Ton-, Bild- und/oder Videoaufnahme 51, 52, 53, 54 erfolgen.

Im Anschluss an der vom Patienten durchgeführten Datenerhebung kann der Arzt, im Rahmen der Kontrollvisite, standardisierte, wiederkehrende Daten 43 in Figur 3 anhand der vorliegenden Erfindung erheben und deren Korrelation mit dem vom Patienten vorhergehend erhobenen Daten bestimmen. Dadurch wird dem Arzt eine neue, technische Möglichkeit 37 - 47 eröffnet, die medizinische Intervention wiederkehrend zu überprüfen, um somit eine genauere und fachlich-spezifischere Nutzen- und Schadenbewertung vorzunehmen 46, unter Einbezug von selbst erfassten Patientendaten (Patient Reported Outcome).

Um die Benutzerfreundlichkeit und Integration in bestehende Prozesse weiter zu fördern, eröffnet die vorliegende Erfindung dem Patienten und dem Arzt die Möglichkeit neben der standardisierten wiederkehrenden Datenerhebung vom Patienten 32 in Figur 2 und vom Arzt 44 in Figur 3 den Fragenkatalog personalisiert zu ergänzen oder zu erweitern, um die Signifikanz der Nutzen- und Schadenbewertung zu erhöhen.

Die vorliegende Erfindung vereinfacht die von Patienten eingenommenen Medikamente 33 in Figur 2 mit den dazugehörigen Dosierungen und Nebenwirkungen (Adverse Event) und die Einhaltung der Medikamenteneinnahme (Compliance) zu dokumentieren. Zunächst muss der Patient in ein prozessorgeneriertes Eingabeformular 33 in Figur 2, welches auf dem Patientendateneingabegerät abgebildet ist, den Namen des Medikaments oder die Barcodenummer eingeben. Erfolgt die Eingabe des Medikamentes in das Eingabeformular über die Barcodenummer, so kann er diese entweder manuell oder anhand eines Barcodelesegerätes, welches mit dem Patientendateneingabegerät über eine standardisierte Schnittstelle wie USB oder RS232, Bluetooth u. ä. angeschlossen oder integriert ist, vornehmen. Erfolgt die Eingabe des Medikamentes in das Eingabeformular über den RFID Chip, so kann er diese anhand eines RFID Lesegerätes vornehmen. Das Patientendateneingabegerät verbindet sich automatisch mit dem Medikamentenartikeldatenbankserver 10 in Figur 1, um die angeforderten Informationen über das exakte Medikament im Profil des Datenbankservers des chronischen Patienten anzuzeigen und abzuspeichern. Falls kein eindeutig zuordenbares Medikament gefunden wird, so sendet der Datenbankserver dem Patientendateneingabegerät eine alphabetisch sortierte Resultatsliste von Medikamenten zu, welches die Suchmerkmale aufweisen. Der Patient wählt manuell das entsprechend eingenommene Medikament mit Angabe der Verpackungsgrösse aus und speichert diese Informationen in seinem Nutzerkonto des Datenbankservers.

Die Nebenwirkungen von Medikamenten können nachträglich über das Patientendateneingabegerät 2 des Patienten 1 dokumentiert werden, indem der Patient über die Verbindung zum Datenbankserver 26 die entsprechende Medikamenteneinnahme in seinem Medikamentenverlauf aufruft und die Nebenwirkungen text- und zeichenbasierend, sowie mit Ton- / Bild-/ Videoaufnahmen einträgt und diese Änderung zu seinem Nutzungskonto hinzufügt und an den Datenbankserver 26 sendet.

Die Übertragung von Daten zu und vom Patientendateneingabegerät und/oder dem Arztdateneingabegerät 20 zum Datenbankserver 26 erfolgt nach Authentifizierung über öffentliche und/oder private Netze in verschlüsselter Form nach einem sicheren Standardverfahren nach dem Stand der Technik.

Das computerimplementierte, interaktive System und Verfahren in Echtzeit zur Nutzen- und Schadenbewertung untersucht und berechnet automatisch 11 die erhobenen Daten, um den Zusatznutzen von Arzneimitteln unter Alltagsbedingungen auszuwerten.

Das System und Verfahren zur Nutzen- und Schadenbewertung von medizinischen Interventionen in der angeführten Erfindung ist ein automatisiertes Echtzeit System und Verfahren. Dies eignet sich insbesondere gut für die Erbringung eines Nachweises mit Betroffenen für den Zusatznutzen von neuen Medikamenten unter echten Alltagsbedingungen, welches rechtlich vorausgesetzt wird.

Die automatisierte Nutzen- und Schadenbewertung wiederum kann dazu führen, dass die Entwicklungen und der Markt- und Vertriebszugang von neuen Medikamenten schneller und umfangreicher bestimmt werden können.

Durch die technische Vernetzung 16, 18, 24, 19 des Patienten und dem behandelnden Arzt können mit der vorliegenden Erfindung, auch pharmakoökonomisch 17, 21, 22 relevante und transparente Daten unter Alltagsbedingungen in Echtzeit gewonnen werden.

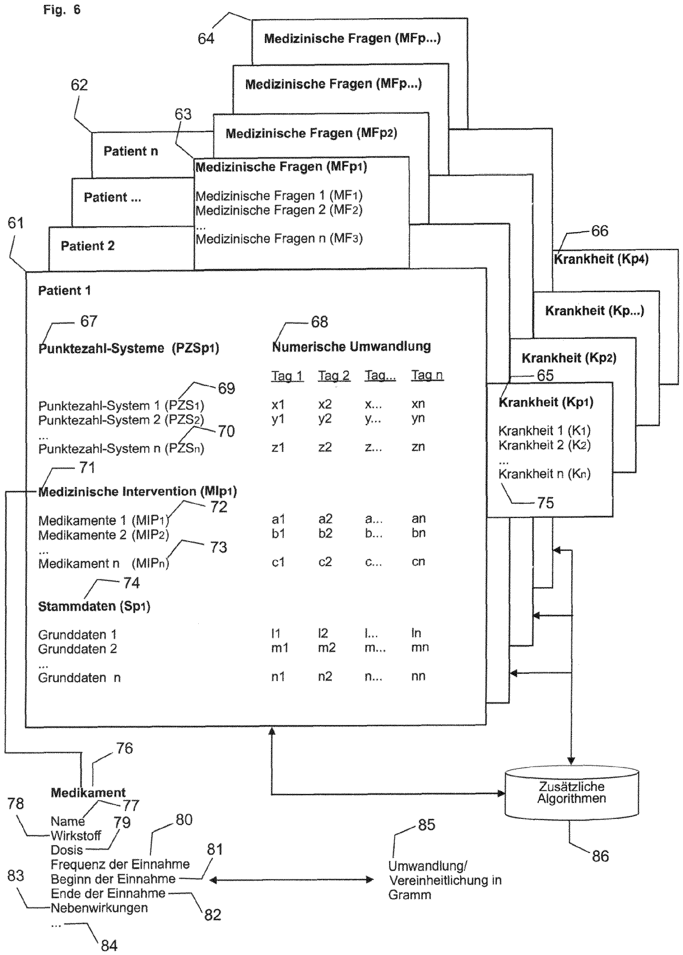

Die vorliegende Erfindung dient der verbesserten Benutzerfreundlichkeit, die dem Patienten die Möglichkeit eröffnet, die Datenerhebung in Abhängigkeit zu seinem spezifischen Krankheitsbild 65, 66, 75 in Figur 6 und Krankheitsstadium durchzuführen. So sieht die vorliegende Erfindung die Eingabemöglichkeit der Daten seitens des Patienten in Abhängigkeit zu seiner Krankheit und der daraus folgenden spezifischen körperlichen Einschränkung. Das heisst, dass die vorliegende Erfindung eine text- und zeichenbasierende Eingabemöglichkeit sowie Ton-/Bild-/Videoaufnahmen 49, 51, 52, 53, 54 in Figur 4 umfasst.

Insbesondere krankheitsbedingte Patienten, die in der Bewegungsfreiheit eingeschränkt sind, können durch die automatisierte Eingabemöglichkeit mittels einem Barcodelesegerät oder anderen elektrooptischen und Bilderkennungssystemen, die Eingabe von Medikamenten 76 in Figur 6 vollständig und fehlerlos eingeben und so die eingenommenen Medikamente 77, 78 dokumentieren. Automatisiert bedeutet, dass der spezifische Datensatz des Medikamentes nach Lesung des codierten Musters der grafischen Vorrichtung wie z. B. Barcodes, den hinterlegten und zugehörigen Wert des Artikels automatisch aus dem Medikamentenartikeldatenbankserver aufrufen und mit Zusatzeingabe von weiteren Parametern, wie Beginn 81 und Ende 82 der Einnahme, Dosierung 79, Häufigkeit 80, Nebenwirkungen 83 und weiteren Parametern 84, vom Patienten mit einem Zeitstempel im Nutzerkonto des Patienten dokumentiert werden.

In wiederkehrenden Datenerhebungen 63, 64 durch Patient und Arzt wird mit dem prozessorgenerierten Eingabeformular der einzelne Patientenzustand numerisch und zeitlich 68 fortlaufend erfasst und im Nutzerkonto 74 des jeweiligen Patienten im Datenbankserver mit Zeitstempel gespeichert, wie in Figur 2 und Figur 3. Der Wert des Arztes kann zusätzlich mit Gewichtungsparametern ergänzt werden.

In wiederkehrenden Datenerhebungen durch den Patienten werden mit dem prozessorgenerierten Eingabeformular die einzelnen Medikamente nach Name, Wirkstoff, Krankheitszugehörigkeit, Dosis, Frequenz der Einnahme, Beginn der Einnahme, Ende der Einnahme und Nebenwirkungen und/oder weiteren Angaben zeitlich fortlaufend erfasst und im Nutzerkonto des jeweiligen Patienten im Datenbankserver mit Zeitstempel gespeichert, wie in Figur 2 und Figur 3.

Mit einer Mehrzahl von international anerkannten Punktezahl-Systemen 67, 69, 70 in Figur 6, die der unterschiedlichen und vielschichtigen Klassifizierung der spezifischen Krankheitsaktivitäten dienen, wird der numerisch hinterlegte und wiederkehrend erhobene Patientenzustand durch Patient und Arzt schliesslich in einer Mehrzahl von einheitlicher Nomenklatur zeitlich fortlaufend beschrieben und gespeichert. Diese Vereinheitlichungen dienen der statistischen Erfassung. Allen Punktezahl-Systemen sind definierte Punktewerte zugeordnet und ihr Ergebnis wird mittels Berechnungsverfahren auf einer festen Skala abgebildet.

Bei der Berechnung 61, 62 der Nutzen- und Schadenbewertung wird in einem ersten Schritt der bemessene Zeitraum definiert, auf den sich die Daten beziehen.

Die Medikamente 71, 72, 73 werden auf Basis der Dosis und Anzahl der Einnahmen aus einer Mehrzahl eingenommener Medikamenten über einen gleitenden Zeitträger in numerische Werte umgewandelt. Konkret wird der erste Wert aus der Frequenz der Einnahmen des Medikamentes berechnet. Der zweite Wert wird als kumulatives Gesamtgewicht der jeweilig eingenommenen Medikamente in Gramm berechnet, wobei andere Dosis Einheiten in ihrer spezifischen Masseinheit automatisch vom Prozessor in Gramm umgerechnet werden 85. Beide Werte und alle weiteren Werte werden jeweils als anteilsmässige und gewichtete Masszahl berechnet und zeitlich fortlaufend in der Datenbank gespeichert. Gibt es nur einen Medikamentenwert, so repräsentiert dieser Gesamthaft die medizinische Intervention.

Der Differenzwert oder das Delta von einem zeitpunktbezogenen Datensatz auf den nächsten vom Mittelwert aus einer Mehrzahl von Punktezahl-Systemen gilt in Verbindung mit den Werten aus der medizinischen Intervention zu setzen. Damit wird die Stärke des Zusammenhangs zwischen den Punktewerten aus den Punktezahl-Systemen und der medizinischen Intervention quantifiziert. Gibt es keinen zeitlich vorausgehenden Datensatz, so ist die Veränderung gleich null (Anfangswert).

Diese Werte, aus einer Mehrzahl von Medikamenten, dienen der numerischen Umwandlung 68 einer medizinischen Intervention (a1, ..., cn). Sind es mehrere Medikamente in derselben Zeitperiode, wird der Einfluss der Veränderung der Punktezahl-Systeme (x1, ..., zn) proportional den einzelnen Medikamenten zugeordnet.

In einem weiteren Schritt wird die Gesamtanzahl der Veränderung nach Medikament oder Wirkstoff als Nutzen (bei positiver Veränderung) oder als Schaden (bei negativer Veränderung) in der Datenbank mit einem numerischen Wert gespeichert und auf einer grafischen Nutzeroberfläche dargestellt. Um die Berechnung nachzuvollziehen, werden alle Medikamente mit Namen sowie Detailangaben aufgelistet, um schliesslich die Nutzen- und Schadenbewertung erstmals in Prozent der gesundheitlichen Verbesserung oder Verschlechterung pro Tag (Verbesserung Lebenszustand) oder in Prozent bis zur Genesung (Verkürzung der Krankheitsdauer) auszudrücken. Weitere Spezifizierung können unternommen werden 86.

Der Arzt kann die Datenaussage zusätzlich durch Hinzunahme der statistischen Berechnungsmodelle 86 weiter verdichten. Er kann auch durch Eingabe der Regressions- und Korrelationsparameter in sein Arztdateneingabegerät die Signifikanz der Nutzen- und Schadenbewertung von medizinischen Interventionen erhöhen. Parameter beinhalten die Spezifizierung der Krankheit und Unterkrankheit (88) nach dem IDC-Katalog der WHO (World Health Organization). Weitere Grunddaten wie Alter, Geschlecht, Herkunft können eingegeben werden. Des Weiteren ist es möglich zusätzliche Parameter über medizinische Interventionen 90 nach Markennamen, Arzneimittelhersteller zwecks pharmakoökonomischer Betrachtungsweisen und/oder weitere Eingrenzungen durch die Punktezahl-Systeme 91 oder Scores (z. B. RADAI, BASDAI, BASFI, CDAI, PASI für Immunologie-Erkrankungen) einzugeben. Darüber hinaus können weitere Parameter sowie Restriktionen, Regeln, Auswertungen und Algorithmen 92 zur zielgenauen, automatisierten Berechnung definiert werden. Dazu gehören zum Beispiel die Evidenzgrade mit Vergleichsmöglichkeiten zur Korrelation von Patienten-Arzteingaben, Populationen, Regressionsanalysen, Sensitivitätsanalysen und Standardabweichungen.

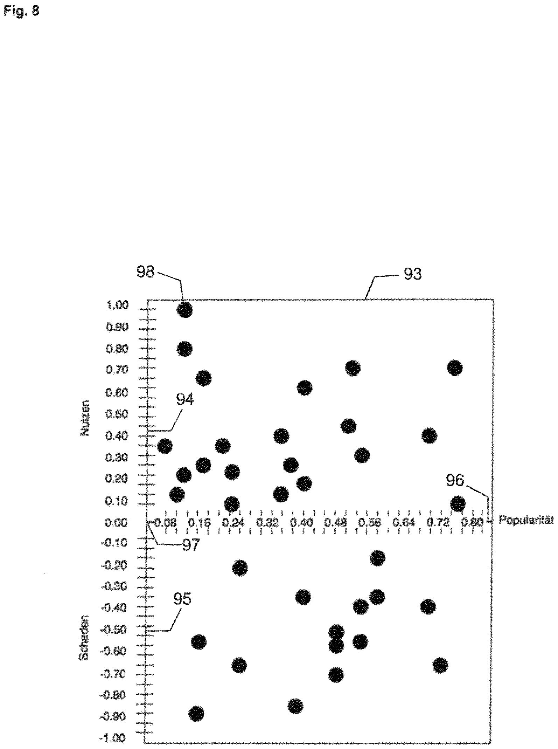

In einem weiteren Schritt wird durch weitere statistische Berechnungsverfahren aus einer Mehrzahl von Nutzern für dieselbe Krankheit oder Krankheitsbild die Nutzen- und Schadenbewertung 93 in Figur 8 durchgeführt und geglättet. Das Ergebnis wird in einem Koordinatensystem dargestellt, wobei die Y-Achse den Nutzen 94 beziehungsweise Schaden 95 und die X-Achse den Bruchteil der Popularität 96 der medizinischen Intervention in Bezug auf alle relevanten Patienteneingaben darstellt. Der Wert null 97 auf der Y-Achse bedeutet weder Nutzen noch Schaden. Der Wert über null auf der Y-Achse bedeutet einen Nutzen, der Wert unter 0 einen Schaden. Der Wert mit dem höchsten Y-Wert ist nach der Nutzen- und Schadenbewertung der Goldstandard 98 einer medizinischen Intervention innerhalb einer Krankheit oder Krankheitsbild, also die beste medizinische Intervention und Methode zur Therapie.

Betrachtet man die aus dem Zusammenspiel der eben, benannten einzelnen, bestehenden Vorteilen, so wird eine bisher fehleranfällige Nutzen- und Schadenbewertung der medizinischen Interventionen im Einsatz von medizinischen Interventionen behoben.

## Patentansprüche

1. Interaktives Computer- und Telekommunikationssystem für Nutzen- und Schadenbewertung medizinischer Interventionen und Medikamenten-Verabreichungen, umfassend:
einen zentralen Computer (15) mit mindestens einem Prozessor und einem Datenbankserver (26) mit Speicher sowie eine Vielzahl von über separate drahtlose Schnittstellen mit dem Computer verbundenen nutzerspezifischen Eingabe- und Anzeigegeräten für eine manuelle Eingabe von Text und Zeichen sowie Ton, Bild, Video für mindestens folgende vier Nutzerkategorien;
• Patienten (1),
• Ärzteschaft (23),
• Arzneimittelhersteller (17),
• Krankenkasse (21),
wobei im Datenbankserver (26) alle relevanten Daten zum auf der Zeitachse lückenlosen Verfolgen von medizinischen Interventionen sowie Medikamenten-Verabreichungen wie auch der Patientenzustände von den Patienten (1) und ihren Ärzten (23) durch manuelle Eingabe als Text und Zeichen, oder durch technische Übermittlung in Form von Daten aller Art wie Ton, Bild, Video aus Mess- und
Aufnahmegeräten über eine Schnittstelle direkt ablegbar sind, und diese Daten automatisiert und technisch für eine Nutzen- und Schadenbewertung verarbeitbar sind und die Daten wie Ton, Bild, Video und generierten Ergebnisse mit individuellen, auf die Nutzerkategorien (1, 23, 17, 21) zugeschnittenen Zugangsberechtigungen entweder personalisiert oder anonymisiert abrufbar sind.

2. Verfahren für die Erstellung von Nutzen- und Schadenbewertungen medizinischer Interventionen und Medikamenten-Verabreichungen mittels eines interaktiven Computer- und Telekommunikations-systems nach Anspruch 1, welches einen zentralen Computer (15) mit mindestens einem Prozessor und einem Datenbankserver (26) mit Speicher sowie eine Vielzahl von über separate drahtlose Schnittstellen mit dem Computer verbundenen nutzerspezifischen Eingabe- und Anzeigegeräten für mindestens folgende vier Nutzerkategorien;
• Patienten (1),
• Ärzteschaft (23),
• Arzneimittelhersteller (17),
• Krankenkasse (21),
umfasst, indem
a) die Patienten oder Ärzte alle medizinischen Indikationen und Medikamenten-Verabreichungen zu diesem betreffenden Patienten mit Angabe von Zeit und qualitativer und quantitativer Spezifikationen in standardisierter Form durch manuelle Eingabe von Text und Zeichen sowie Ton, Bild, Video über ihre Eingabe- und Anzeigegeräte an den Zentralcomputer übermitteln oder in Form von Daten aller Art wie Ton, Bild, Video aus Mess- und Aufnahmegeräten über eine Schnittstelle direkt übermitteln,
b) die abgelegten medizinischen Indikationen und Medikamenten-Verabreichungen sowie Patientenzustände automatisiert und technisch für eine Nutzen- und Schadenbewertung verarbeitet werden,
c) die verarbeiteten Daten und damit generierten Ergebnisse spezifisch zugangsberechtigten Nutzern personalisiert oder anonymisiert zugänglich gemacht werden.

3. Interaktives Computer- und Telekommunikationssystem für Nutzen- und Schadenbewertung medizinischer Interventionen und Medikamenten-Verabreichungen nach Anspruch 1, **gekennzeichnet durch** die Merkmale A-D zur Durchführung der Schritte e-t:
A. der zentrale Computer (15) schliesst einen programmierten Computer Prozessor ein, dafür geeignet, die nachfolgenden Schritte i, j, k, l, m, n, o, p, q, r, s und t durchzuführen;
B. der Datenbankserver (26) enthält einen Speicher, der in elektronischer Verbindung mit dem Prozessor (15) steht, dafür geeignet, die nachfolgenden Schritte e, f, g, h, i, j, k, o und q durchzuführen;
C. die nutzerspezifischen Eingabe- und Anzeigegeräte sind einerseits Patientendateneingabe- und -anzeigegeräte (2), die in elektronischer Verbindung (24) mit dem Prozessor (15) stehen, dafür geeignet, die nachfolgenden Schritte i, s und t durchzuführen;
D. die nutzerspezifischen Eingabe- und Anzeigegeräte sind andrerseits Arztdateneingabe- und -anzeigegeräte (20), die in elektronischer Verbindung (16) mit dem Prozessor (15) stehen; und mit denen je nach Nutzer, das heisst entweder vom Arzt, dem Arzneimittelhersteller oder der Krankenkasse, die nachfolgenden Schritte j, s und t auf dem Computer (15) durchführbar sind;
e. Speicherung von Krankheiten (K₁, K₂, ... Kₙ) im Speicher des Datenbankservers;
f. Speicherung einer Liste von international anerkannten medizinisch-relevanten Fragen (MF₁, MF₂, ... MFₙ), sodass für jede spezifisch medizinische Fragestellung eine Zuordnung zur Krankheit gespeichert wird;
g. Speicherung von Formeln nach international anerkannten Punktezahl-Systemen, sodass für jede Variable der Rechenformel der Bezug zu der medizinisch-relevanten Frage gespeichert wird;
h. Speicherung von Medikamenten mit Angaben zu Namen, Wirkstoff, Verpackungsgrösse und Verpackungseinheit und weitere Informationen über das Medikament zusammenfassend als medizinische Interventionen (MI₁, MI₂, ..., MIₙ) im Speicher des Datenbankservers (26), so dass für jeden gespeicherten Patienten, eine oder mehrere gespeicherte Krankheiten, eine oder mehrere gespeicherte Krankheitswerte und eine oder mehrere gespeicherte medizinische Interventionen mit numerischen Antwortwerten gespeichert sind;
i. Manuelle Eingabe von Text und Zeichen sowie Ton, Bild, Video eines Patienten aus einer Mehrzahl von Patienten, Patientendatenangaben von Grund- und Vitalparameter sowie Informationen über die Einnahme von Medikamenten in den Speicher des Datenbankservers (26) einzugeben unter Nutzung eines Patientendateneingabegerätes (2), das in elektronischer Verbindung (24) mit dem Prozessor (15) steht und um fortlaufend weitere spezifische medizinische Daten wie Lebensqualitäts- und Gesundheitsbewertungsparameter sowie weitere Einnahmen von Medikamenten nach Krankheiten in den Datenbankserver (26) einzugeben;
j. Manuelle Eingabe von Text und Zeichen sowie Ton, Bild, Video eines Arztes (23) aus einer Mehrzahl von Ärzten, Patientendatenangaben von Vitalparameter in den Speicher des Datenbankservers (26) einzugeben unter Nutzung eines Arztdateneingabegerätes (20), das in elektronischer Verbindung (16) mit dem Computer (15) steht;
k. Berechnung von zeitlich fortlaufenden Punktezahl-Systemwerten (PZS₁, PZS₂, ..., PZSₙ) auf Basis der Schritte i. und j. und Speichern der Ergebnisse im Speicher des Datenbankservers;
l. Berechnung der gesamten Anzahl der Einnahmen nach Stück oder Einheit von Medikament pro zeitpunktbezogenen Datensatz;
m. Berechnung des gesamten Gewichtes der eingenommenen Medikamente pro zeitpunktbezogenen Datensatz, falls nötig Umwandlung vom jeweiligen, spezifischen Eigengewicht in Gramm-Einheiten zur genaueren Vergleichbarkeit;
n. Berechnung der medizinischen Intervention pro Medikament in numerische Werte pro zeitpunktbezogenen Datensatz als anteilsmässige und gewichtete Masszahl aus Schritt I. und m.;
o. Vergleich der zeitlich fortlaufenden Veränderung pro zeitpunktbezogenen Datensatz der Punktezahl-Systemwerte in Abhängigkeit mit den eingenommenen Medikamenten gemäss Schritt n. auf proportionaler Basis und Speichern der Ergebnisse im Speicher des Datenbankservers;
p. Definition des Bemessungszeitraumes **durch** den Nutzer;
q. Berechnung **durch** Verwenden des Prozessors (15), um auf Basis einer Mehrzahl von Patienten (1) für dieselbe Krankheit den geglätteten Mittelwert für die medizinische Intervention zu bewerten **durch** Angabe in Prozent der gesundheitlichen Verbesserung oder Verschlechterung pro Tag oder in Prozent bis zur Genesung den Nutzen und Schaden der einzelnen Medikamente oder in Prozent der Verkürzung der Krankheitsdauer oder in Prozent der Verbesserung bzw. Verschlechterung der Lebensqualität und Speichern des Ergebnisses im Speicher des Datenbankservers;
r. Berechnung der Nutzen- und Schadenbewertung **durch** Hinzunahme weitere statistischen Berechnungsmodelle sowie gewichtete Arztdateneingaben nach Patient (1) und Spezifikationen der zu kontrollierenden Teilpopulationen sowie Bestimmung der medizinischen Intervention nach Medikamentenname oder Wirkstoff;
s. Anzeigen der Daten der Nutzen- und Schadenbewertung auf einer grafischen Oberfläche in Form eines Koordinatensystems, welches eine Skalierung für den Nutzen bzw. Schaden auf der Ordinate und eine Skalierung für die Popularität auf der Abszisse anzeigt (93);
t. Anzeigen der Daten der Nutzen- und Schadenbewertung in Tabellenform mit allen Einzeldaten;

4. Verfahren für die Erstellung von Nutzen- und Schadenbewertungen medizinischer Interventionen und Medikamenten-Verabreichungen nach Anspruch 2, **dadurch gekennzeichnet, dass** folgende Schritte durchgeführt werden:
e. Speicherung von Krankheiten (K₁, K₂, ... Kₙ) im Speicher des Datenbankservers (26);
f. Speicherung einer Liste von international anerkannten medizinisch-relevanten Fragen (MF₁, MF₂, ... MFₙ), sodass für jede spezifisch medizinische Fragestellung eine Zuordnung zur Krankheit gespeichert wird;
g. Speicherung von Formeln nach international anerkannten Punktezahl-Systemen, so dass für jede Variable der Rechenformel der Bezug zu der medizinisch-relevanten Frage gespeichert wird;
h. Speicherung von Medikamenten mit Angaben zu Namen, Wirkstoff, Verpackungsgrösse und Verpackungseinheit und weitere Informationen über das Medikament zusammenfassend als medizinische Intervention (MI₁, MI₂, ..., MIₙ) im Speicher des Datenbankservers (26), sodass für jeden gespeicherten Patienten (1), eine oder mehrere gespeicherte Krankheit, eine oder mehrere gespeicherte Krankheitswerte und eine oder mehrere gespeicherte medizinische Intervention mit numerischen Antwortwerten gespeichert sind;
i. Aufforderung an einen Patienten (1), aus einer Mehrzahl von Patienten, um Patientendateneingaben von Grund- und Vitalparameter sowie Informationen über die Einnahme von Medikamenten in den Speicher des Datenbankservers (26) einzugeben unter Nutzung eines Patientendateneingabegerätes (2), das in elektronischer Verbindung (24) mit dem Prozessor (15) steht und um fortlaufend weitere spezifische medizinische Daten wie Lebensqualitäts- und Gesundheitsbewertungsparameter nach Krankheit in den Datenbankserver (26) einzugeben;
j. Aufforderung an einen Arzt (23), aus einer Mehrzahl von Ärzten, Patientendatenangaben von Vitalparameter in den Speicher des Datenbankservers (26) einzugeben unter Nutzung eines Arztdateneingabegerätes (20), das in elektronischer Verbindung (16) mit dem Prozessor (15) steht;
k. Berechnung von zeitlich fortlaufenden Punktezahl-Systemwerten (PZS₁, PZS₂, ..., PZSₙ) auf Basis der Schritte i. und j. und Speichern der Ergebnisse im Speicher des Datenbankservers;
l. Berechnung der gesamten Anzahl der Einnahmen nach Stück oder Einheit von Medikament pro zeitpunktbezogenen Datensatz;
m. Berechnung des gesamten Gewichtes der eingenommenen Medikamente pro zeitpunktbezogenen Datensatz, falls nötig Umwandlung vom jeweiligen, spezifischen Eigengewicht in Gramm-Einheiten zur genaueren Vergleichbarkeit;
n. Berechnung der medizinischen Intervention pro Medikament in numerische Werte pro zeitpunktbezogenen Datensatz als anteilsmässige und gewichtete Masszahl aus Schritt I. und m.;
o. Vergleich der zeitlich fortlaufenden Veränderung pro zeitpunktbezogenen Datensatz der Punktezahl-Systemwerte in Abhängigkeit mit den eingenommenen Medikamenten gemäss Schritt n. auf proportionaler Basis und Speichern der Ergebnisse im Speicher des Datenbankservers;
p. Definition des Bemessungszeitraumes;
q. Berechnung durch Verwenden des Prozessors (15), um auf Basis einer Mehrzahl von Patienten (1) für dieselbe Krankheit den geglätteten Mittelwert für die medizinische Intervention zu bewerten durch Angabe in Prozent der gesundheitlichen Verbesserung oder Verschlechterung pro Tag oder in Prozent bis zur Genesung den Nutzen und Schaden der einzelnen Medikamente oder in Prozent der Verkürzung der Krankheitsdauer oder in Prozent der Verbesserung bzw. Verschlechterung der Lebensqualität und Speichern des Ergebnisses im Speicher des Datenbankservers;
r. Berechnung der Nutzen- und Schadenbewertung durch Hinzunahme weiterer statistischer Berechnungsmodelle sowie gewichteter Arztdateneingaben nach Patient (1) und Spezifikationen der zu kontrollierenden Teilpopulationen sowie Bestimmung der medizinischen Intervention nach Medikamentenname oder Wirkstoff;
s. Anzeigen der Daten der Nutzen- und Schadenbewertung auf einer grafischen Oberfläche mit einem Koordinatensystem (93);
t. Anzeigen der Daten der Nutzen- und Schadenbewertung in Tabellenform mit allen Einzeldaten.
